(19) Europäisches Patentamt
European Patent Office
Office européen des brevets

(11) EP 4 570 252 A1

(12) EUROPEAN PATENT APPLICATION
published in accordance with Art. 153(4) EPC

(43) Date of publication:
**18.06.2025 Bulletin 2025/25**

(21) Application number: **23852462.3**

(22) Date of filing: **02.08.2023**

(51) International Patent Classification (IPC):
***A61K 31/7004** (2006.01)* ***A61P 13/02** (2006.01)*
***A61P 31/00** (2006.01)*

(52) Cooperative Patent Classification (CPC):
**A61K 31/7004; A61P 13/02; A61P 31/00**

(86) International application number:
**PCT/JP2023/028305**

(87) International publication number:
**WO 2024/034495 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.08.2022 JP 2022127106**

(71) Applicant: **Sunus Co., Ltd.**
**Kagoshima-shi, Kagoshima 891-0196 (JP)**

(72) Inventor: **YOSHINAGA, Kazuhiro**
**Kagoshima-shi, Kagoshima 891-0196 (JP)**

(74) Representative: **Kraus & Lederer PartGmbB**
**Thomas-Wimmer-Ring 15**
**80539 München (DE)**

# (54) URINARY TRACT INFECTION PREVENTION/TREATMENT AGENT CONTAINING 1-DEOXYMANNOSE

(57) There is provided a preventive and therapeutic agent for urinary tract infection containing 1-deoxymannose.

Fig. 1

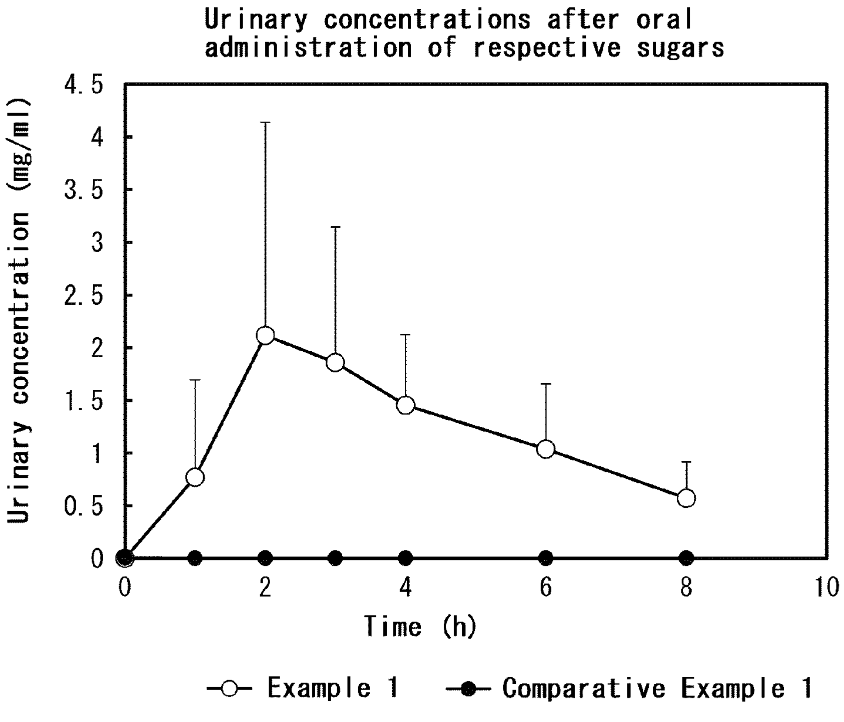




Processed by Luminess, 75001 PARIS (FR)

## Description

Technical Field:

The present invention relates to a preventive and therapeutic agent for urinary tract infection containing 1-deoxymannose.

Background Art:

A urinary tract infection is a disease caused by the presence of pathogenic microorganisms in the urinary tract, such as the renal pelvis, ureter, urethra, and bladder, and it is classified into cystitis and pyelonephritis according to the site of infection. In many cases, a urinary tract infection is caused by bacterial pathogenic microorganisms, most of which are regarded as uropathogenic Escherichia coli (UPEC). A urinary tract infection is usually treated with antibiotics, which however leads to the development of antibiotic-resistant bacteria. Another known treatment is the intake of mannose in the form of a supplement.

A urinary tract infection caused by UPEC develops when Fim H lectin of UPEC binds to mannose residues at the end of sugar chains protruding from the urothelial cells. The Fim H lectin also binds to mannose. Thus, when free mannose is present in urine, UPEC becomes more likely to bind to this mannose and less likely to bind to the urothelial cells, whereby a urinary tract infection can be controlled. UPEC that fails to bind to the urinary tract is eliminated in urine from the body.

Mannose has been orally tested in human trials with the following results reported: Mannose derived from plant hemicellulose or a mixture of mannose and a cranberry component is contained in a preventive and improving agent for urinary tract infection (Patent Documents 1 and 2). Further, mannose is reportedly effective in the prevention of cystitis cases (Non-patent Document 1). Meanwhile, considering the report that only approximately 0.1% of orally administered mannose reaches the urinary tract (Non-patent Document 2), mannose has limited effects in preventing and treating urinary tract infection.

For the purpose of discovering a substance that exhibits stronger anti-adhesion activity than mannose, many mannose derivatives have been developed (Non-patent Document 3). However, considering the fact that mannose derivatives are substances obtained by chemical modification of mannose, the rate of small-intestinal absorption of mannose derivatives administered orally to humans is presumed to be even lower than that of mannose, which would also make it difficult for such mannose derivatives to reach the urinary tract. In addition, mannose derivatives have not been evaluated in clinical trials as to how much of mannose derivatives administered orally to humans reaches the urinary tract and how effective they are for the prevention and treatment of urinary tract infection; thus, their effect is questionable.

Further, since urinary tract infection has a high rate of recurrence, more effective methods of prevention and treatment are required.

Prior Art Documents:

Patent Documents:

Patent Document 1: JP-A-2011-219469
Patent Document 2: JP-A-2012-171899

Non-patent Documents:

Non-patent Document 1: Antibiotics 2021, 10, 373.
Non-patent Document 2: Luminacoids Research, Vol. 22, No. 2 (2018)
Non-patent Document 3: Molecules 2018, 23, 1641
Non-patent Document 4: IUCrJ (2017) 4, 7-23

Summary of the Invention:

Problems to be solved by the Invention:

As described above, many reports have been published on substances, such as mannose and mannose derivatives, that bind to Fim H lectin. However, no or insufficient assessment has been made of the effects of these

substances on humans. Under the circumstances, it is required to provide a preventive and therapeutic agent for urinary tract infection that can be administered safely to humans and can reach the urinary tract at an effective concentration to exhibit anti-adhesion activity against UPEC.

An object of the present invention is to provide a highly effective preventive and therapeutic agent for urinary tract infection that can be administered safely to humans. In an effort to find materials that meet these requirements, the inventors have discovered that 1-deoxymannose administered to humans is excreted in urine at a high rate; thus, the present invention has been completed.

Means for solving the Problems:

The present invention provides a preventive and therapeutic agent for urinary tract infection containing 1-deoxymannose.

In the preventive and therapeutic agent for urinary tract infection, the 1-deoxymannose is preferably detected in human urine after administration of the agent.

In the preventive and therapeutic agent for urinary tract infection, urinary 1-deoxymannose concentration after administration of the agent is preferably higher than urinary mannose concentration after administration of mannose.

In the preventive and therapeutic agent for urinary tract infection, a urine volume after administration of the agent is preferably larger than a urine volume after administration of mannose.

The preventive and therapeutic agent for urinary tract infection is preferably administered orally.

Effect of the Invention:

The preventive and therapeutic agent for urinary tract infection according to the present invention contains 1-deoxymannose so that it can reach the urinary tract at an effective concentration to exhibit anti-adhesion activity against UPEC, resulting in a high preventive and therapeutic effect against urinary tract infection.

Brief Description of the Drawings:

[Fig. 1]: a graph showing urinary 1-deoxymannose concentration and urinary mannose concentration after oral administration of the respective sugars;
[Fig. 2]: a graph showing the urine volume after oral administration of each of the sugars; and
[Fig. 3]: a graph showing the utilization of sugars by Escherichia coli.

Mode for Carrying out the Invention:

1-deoxymannose, also referred to as 1,5-anhydro-D-mannitol, is a deoxy form of mannose where the oxygen atom of the hydroxy group at the 1-position is removed. 1-deoxymannose is known as a metabolite of 1,5-anhydro-D-fructose (1,5-AF). The chemical structural formula of 1-deoxymannose is shown below.

[Chemical Formula 1]

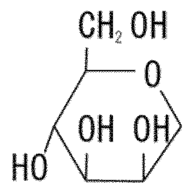

1-deoxymannose

1-deoxymannose for use in the present invention can be synthesized by reducing 1,5-anhydro-D-fructose (1,5-AF). The detection and determination of 1-deoxymannose can be performed by high performance liquid chromatography (HPLC).

In the present invention, when 1-deoxymannose and mannose are administered in the same dose, urinary 1-deoxymannose concentration after administration of 1-deoxymannose is preferably higher than urinary mannose

administration after administration of mannose.

Further, when 1-deoxymannose and mannose are administered in the same dose, the urine volume after administration of 1-deoxymannose is preferably larger than the urine volume after administration of mannose.

The route of administration can be, for example: oral; intravenous, subcutaneous or intraperitoneal by injection, drip infusion, etc. for direct administration into the body; or topical. For ease of administration for patients, oral administration is preferred.

The preventive and therapeutic agent may come in any form, such as tablets, fine powders, capsules, drops, powders, granules, or an injectable form; the form of the agent is not particularly limited. The preventive and therapeutic agent can also contain components necessary for the formulation, such as a pharmaceutical carrier, an excipient, and a stabilizer.

Further, the preventive and therapeutic agent of the present invention can also contain other preventive and therapeutic agents for urinary tract infection (such as cranberries, lactic acid bacteria, polyphenols and ascorbic acid), other pharmacological components, or nutritional components such as glucose.

Hereinafter, the present invention will be described in further detail by way of Examples. The present invention is not limited to these Examples.

Examples:

<Sample preparation>

1-deoxymannose for use in Examples was synthesized as follows: An aqueous 1,5-AF solution was hydrogenated in the presence of a Raney nickel catalyst, and the resultant solution containing a reduced product of 1,5-AF was concentrated and crystallized, whereby a 1-deoxymannose sample was obtained. The thus-obtained sample was confirmed to be of 99% purity or more by HPLC. This 1-deoxymannose sample was used as a test sample.

In Comparative Examples, D-Mannose (pure powder) manufactured by NOW Foods was used as a mannose sample. This mannose sample was confirmed to be of 99% purity or more by HPLC. This mannose sample was used as a test sample.

<HPLC analysis conditions>

HPLC devices: PU2089 plus, AS2055 plus and RI2031 plus (series number) manufactured by JASCO Corporation; MODEL 556 manufactured by GL Sciences Inc.

Columns: MITSUBISHI MCI GEL CK08S; two columns coupled together
Oven temperature: 60°C
Eluent: Water
Detector: Differential refractometer
Flow rate: 1 ml/minute
Injection volume: 100 μL

The determination of 1-deoxymannose was carried out using a calibration curve produced by using the 1-deoxymannose prepared as above. The determination of mannose was carried out using a calibration curve produced by using special grade mannose as a reagent.

<Safety evaluation of 1-deoxymannose>

1,5-AF was orally administered to a human, followed by measurement of urinary metabolites. As a result, approximately 5% of the administered 1,5-AF was recovered in urine as 1-deoxymannose. It is presumed that the orally administered 1,5-AF was reduced in the body to form 1-deoxymannose as a product. As such, the 1,5-AF administration test serves as a safety evaluation test of 1-deoxymannose as a metabolite.

<Example 1>

1g of 1-deoxymannose was dissolved in water to prepare a 20% w/w solution as a sample for oral administration. Urine was collected from six human subjects before administration of the sample. The total amount of sample was administered orally to each of the subjects, and the total amount of urine was collected one, two, three, four, six and eight hours after the administration. The urine collected at each of the points in time was subjected to volume measurement, and then filtered through a membrane filter with a pore size of 0.2 μm, followed by measurement of urinary 1-deoxymannose

concentration by HPLC analysis.

<Comparative Example 1>

Mannose, instead of 1-deoxymannose, was orally administered, followed by collection of urine, in the same manner as in Example 1.

<Urinary 1-deoxymannose concentration>

Fig. 1 shows the results of measuring urinary 1-deoxymannose concentration at the respective points in time after administration of the sample containing 1-deoxymannose in Example 1, and urinary mannose concentration at the respective points in time after administration of the sample containing mannose in Comparative Example 1. Each of the urinary concentrations at each of the points in time is a simple average of the urinary concentrations of the six subjects.

As shown in Fig. 1, 1-deoxymannose was detected from the urine of all the subjects collected one to eight hours after administration. The concentration of 1-deoxymannose was the highest with a value of 2.12 mg/ml two hours after administration. In contrast, mannose was not detected from any of the subjects. This is presumed to be because the concentration of mannose was below the detection limit of HPLC, which was 0.1 mg/ml. Additionally, urinary mannose content was also measured by D-Mannose/D-Fructose/D-Glucose Kit (Megazyme Ltd.; detection limit: 10 μg/ml or less) using urine supernatant obtained after centrifugation (at 10,000 g for 5 minutes) of the urine; however, mannose was still not detected. This proved that 1-deoxymannose reached the urinary tract at a higher concentration than mannose and, thus, exhibits much higher anti-infection activity against UPEC than mannose.

The dissociation constant ($K_D$) of 1-deoxymannose against Fim H lectin is reportedly 1125 nM (185 ng/ml) (Non-patent document 4). However, the highest urinary 1-deoxymannose concentration observed two hours after administration was about 11,000 times as large as the $K_D$ value of 1-deoxymannose. Thus, it was proved that orally administered 1-deoxymannose exhibits higher anti-adhesion activity against Escherichia coli.

<Urine volume>

Fig. 2 shows the results of measuring the urine volume at the respective points in time after administration of the sample containing 1-deoxymannose in Example 1, and the urine volume at the respective points in time after administration of the sample containing mannose in Comparative Example 1. In Fig. 2, the vertical axis indicates the percentage of the urine volume after administration of 1-deoxymannose in Example 1 relative to the urine volume after administration of mannose in Comparative Example 1 taken as 100%, at the respective points in time.

The results show that the 1-deoxymannose administration group had a larger volume of urine than the mannose administration group at all the points in time. Considering that UPEC is eliminated in urine from the body, a larger urine volume leads to a lower risk of becoming infected with UPEC. It is thus presumed that the administration of 1-deoxymannose not only inhibits UPEC infection due to 1-deoxymannose excreted in urine, but also helps UPEC to be eliminated from the body effectively in a larger volume of urine.

<Urinary recovery rate>

The urinary recovery rate of 1-deoxymannose was determined by dividing the amount of recovered 1-deoxymannose, which was obtained from the collected urine volume and the urinary 1-deoxymannose concentration, by the dose. The urinary recovery rate is expressed by the following formula:

$$\text{Urinary recovery rate [\%]} = ((V_1 \times C_1) + (V_2 \times C_2) + (V_3 \times C_3) + (V_4 \times C_4) + (V_6 \times C_6) + (V_8 \times C_8))/M \times 100$$

$V_n$: Urine volume [ml] n hour(s) after administration
$C_n$: Urinary 1-deoxymannose concentration [mg/ml] n hour(s) after administration
M: Dose of 1-deoxymannose (= 1000 mg)

For up to eight hours after administration, the urinary recovery rate of 1-deoxymannose, when calculated as above, was as extremely high as 43.3±9.03%.

<Example 2 and Comparative Examples 2 and 3>

The presence of glucose in the urine or urinary tract of diabetics, etc. is reported to increase the likelihood of bacterial growth as compared with the absence of glucose in the urinary tract, because such glucose serves as a bacterial energy source. On this account, sugar to be administered for the purpose of preventing urinary tract infection is desirably less likely to be utilized by bacteria when it reaches the urinary tract.

In order to evaluate the utilization of 1-deoxymannose by Escherichia coli (NBRC 3301), the following experiment was conducted.

M9 minimal medium (obtained by adding $MgSO_4$ and $CaCl_2$ to Difco™ M9 Minimal Salt) containing 2 ml of 1% glucose was used. Escherichia coli (NBRC 3301) was subjected to shaking at 37°C for 17 hours to prepare a culture solution. 10 μL of the culture solution was added to 2 ml of M9 minimal media containing 1% 1-deoxymannose, 1% mannose, and 1% glucose, respectively, followed by shaking culture at 37°C. During culture, 100 μL of the culture solution was serially sampled and measured for its turbidity (600 nm) using a cell with an optical path length of 1 cm. The growth of Escherichia coli was evaluated based on increased turbidity. The results of the measurement are shown in Fig. 3, in which Example 2, Comparative Example 2, and Comparative Example 3 correspond to the culture solution containing 1-deoxymannose, the culture solution containing mannose, and the culture solution containing glucose, respectively.

As compared to the culture solutions containing mannose and glucose, respectively, the culture solution containing 1-deoxymannose did not have increased turbidity. Considering that increased turbidity indicates increased growth of Escherichia coli, it was found that 1-deoxymannose was less likely to be utilized by Escherichia coli (NBRC 3301) than glucose and mannose. This proves that 1-deoxymannose is a sugar that is suitable for use in preventive and therapeutic agents for urinary tract infection.

## Claims

1. A preventive and therapeutic agent for urinary tract infection containing 1-deoxymannose.

2. The preventive and therapeutic agent for urinary tract infection according to claim 1, wherein the 1-deoxymannose is detected in human urine after administration of the agent.

3. The preventive and therapeutic agent for urinary tract infection according to claim 1 or 2, wherein urinary 1-deoxymannose concentration after administration of the agent is higher than urinary mannose concentration after administration of mannose.

4. The preventive and therapeutic agent for urinary tract infection according to claim 1 or 2, wherein a urine volume after administration of the agent is larger than a urine volume after administration of mannose.

5. The preventive and therapeutic agent for urinary tract infection according to claim 1 or 2, being administered orally.

Fig. 1

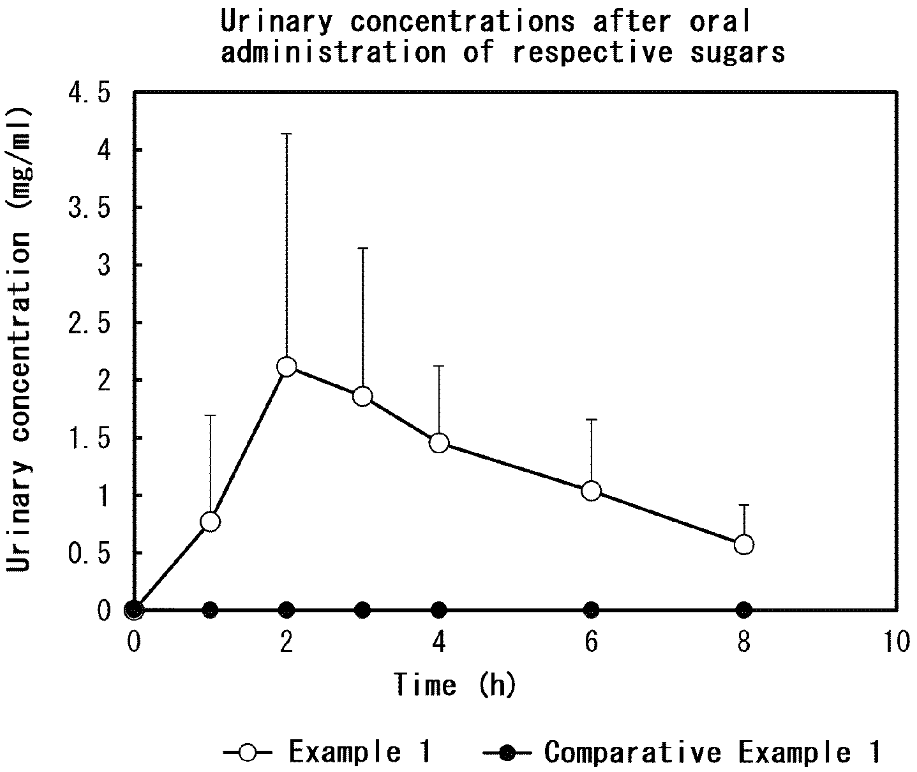
Urinary concentrations after oral administration of respective sugars
Urinary concentration (mg/ml)
4.5
4
3.5
3
2.5
2
1.5
1
0.5
0
0
2
4
6
8
10
Time (h)
Example 1
Comparative Example 1

Fig. 2

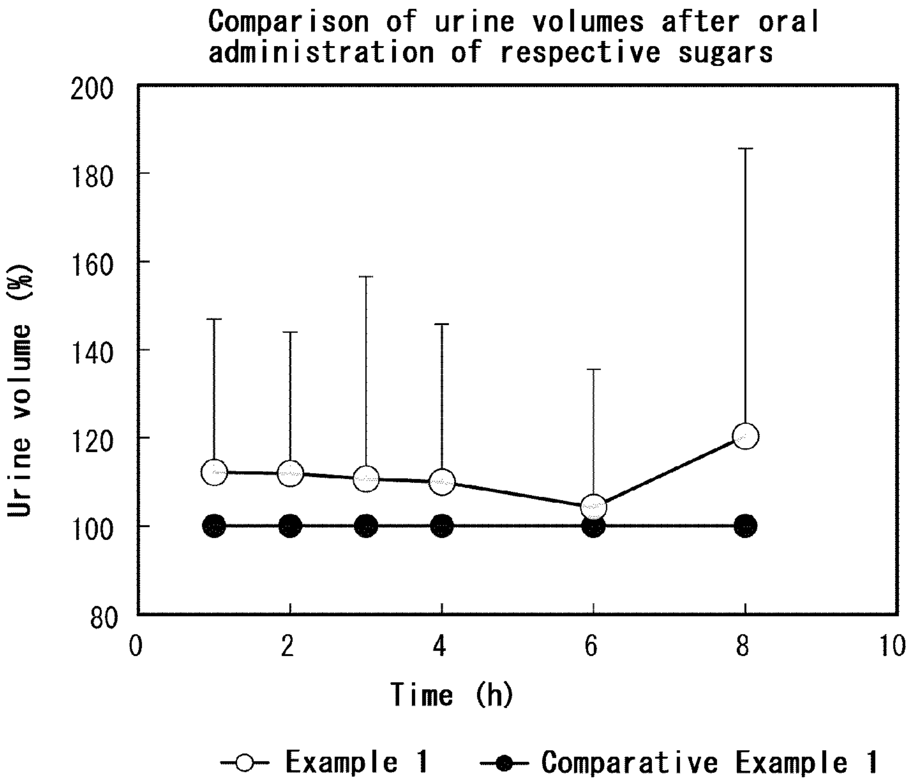
Comparison of urine volumes after oral administration of respective sugars
Urine volume (%)
200
180
160
140
120
100
80
0
2
4
6
8
10
Time (h)
Example 1
Comparative Example 1

Fig. 3

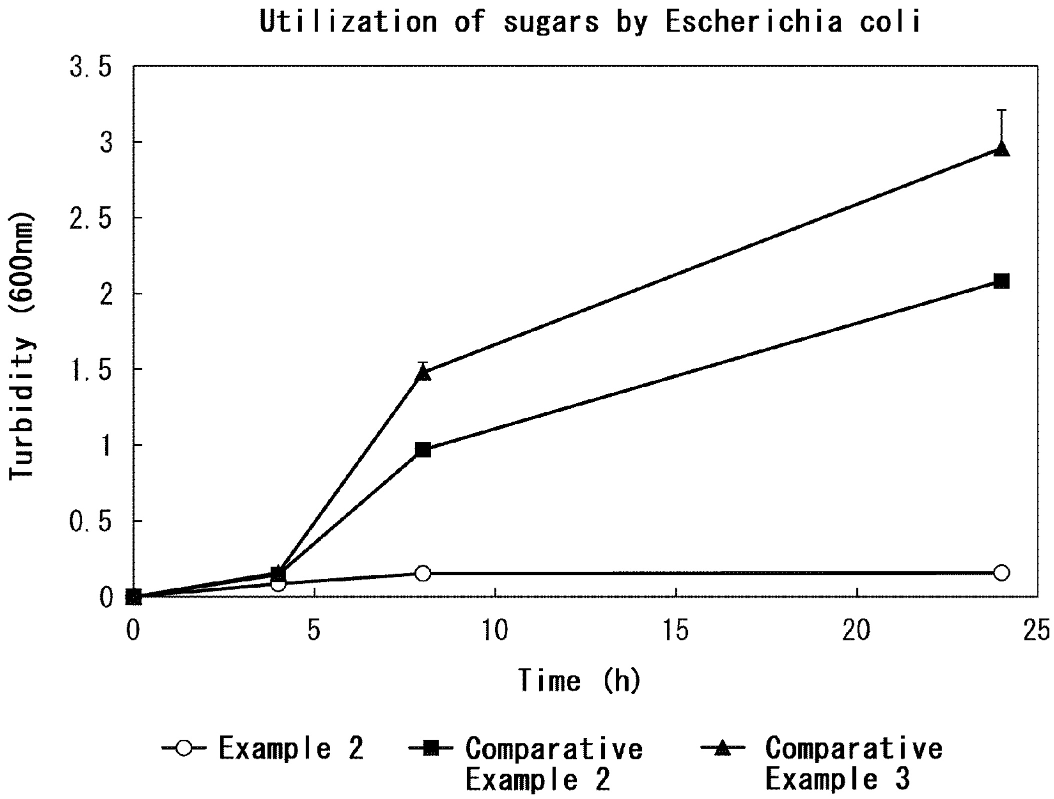
Utilization of sugars by Escherichia coli
3.5
3
2.5
2
1.5
1
0.5
0
Turbidity (600nm)
0
5
10
15
20
25
Time (h)
Example 2
Comparative Example 2
Comparative Example 3

## INTERNATIONAL SEARCH REPORT

International application No.
**PCT/JP2023/028305**

**A. CLASSIFICATION OF SUBJECT MATTER**

***A61K 31/7004***(2006.01)i; ***A61P 13/02***(2006.01)i; ***A61P 31/00***(2006.01)i
FI: A61K31/7004; A61P13/02; A61P13/02 105; A61P31/00

According to International Patent Classification (IPC) or to both national classification and IPC

**B. FIELDS SEARCHED**

Minimum documentation searched (classification system followed by classification symbols)

A61K31/7004; A61P13/02; A61P31/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY/MEDLINE/EMBASE/BIOSIS (STN); JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br><br>Y | FIEGE, Brigitte et al. The Tyrosine Gate of the Bacterial Lectin FimH: A Conformational Analysis by NMR Spectroscopy and X-ray Crystallography, ChemBioChem, 2015, Vol. 16, pp. 1235-1246, ISSN 1439-4227<br>in particular, abstract, p.1235, left column, line 1 to right column, line 16, p. 1238, right column, lines 47-49, table 1, compound 10 | 1-4<br><br>5 |
| Y | JP 2011-219469 A (UNITIKA LTD.) 04 November 2011 (2011-11-04)<br>claims 1, 6, paragraphs [0001], [0024]-[0026], [0035]-[0043], examples 1-6 | 5 |
| Y | JP 2012-171899 A (UNITIKA LTD.) 10 September 2012 (2012-09-10)<br>claims 1, 6-7, paragraphs [0001], [0032]-[0034], [0039]-[0063], examples 1-8 | 5 |
| Y | JP 2015-78151 A (NATIONAL UNIVERSITY CORPORATION NAGOYA UNIVERSITY) 23 April 2015 (2015-04-23)<br>claims 1-2, 5, paragraphs [0022], [0026], [0027], examples | 5 |
| Y | US 2004/0147459 A1 (ONEAL, Joseph) 29 July 2004 (2004-07-29)<br>claims 1-2, 15, paragraph [0017] | 5 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 October 2023** | **24 October 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/028305**

| Patent document cited in search report | Publication date (day/month/year) | Patent family member(s) | Publication date (day/month/year) |
|---|---|---|---|
| JP 2011-219469 A | 04 November 2011 | (Family: none) | |
| JP 2012-171899 A | 10 September 2012 | (Family: none) | |
| JP 2015-78151 A | 23 April 2015 | (Family: none) | |
| US 2004/0147459 A1 | 29 July 2004 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011219469 A
- JP 2012171899 A


### Non-patent literature cited in the description


- *Antibiotics*, 2021, vol. 10, 373
- *Luminacoids Research*, 2018, vol. 22 (2)
- *Molecules*, 2018, vol. 23, 1641
- *IUCrJ*, 2017, vol. 4, 7-23